# EUROPEAN PATENT SPECIFICATION

(11) **EP 3 044 203 B1**
(45) Date of publication and mention of the grant of the patent: **05.09.2018**
(21) Application number: 14786314.6
(22) Date of filing: 10.09.2014
(51) Int. Cl.: C07C 227/42, C07C 229/64

(54) **PROCESS FOR THE PRODUCTION OF HIGH-DENSITY MESALAMINE**
VERFAHREN ZUR HERSTELLUNG VON HOCHDICHTEM MESALAMIN
PROCÉDÉ POUR LA PRODUCTION DE MÉSALAMINE HAUTE DENSITÉ

(30) Priority: 10.09.2013 IT MI20131497
(43) Date of publication of application: 20.07.2016
(73) Proprietor: ERREGIERRE S.p.A., 24060 San Paolo D'Argon (Bergamo) (IT)
(72) Inventor: FERRARI, Massimo, I-24069 Cenate Sotto (IT); GHEZZI, Emanuele, I-24060 Montello (IT); DE LUCA, Florindo, I-20043 Arcore (IT)
(74) Representative: Coppo, Alessandro
(86) International application number: PCT/IB2014/064370
(87) International publication number: WO 2015/036920

(56) References cited:
- WO-A1-2008/044099
- US-A1- 2013 225 539
- Z. BANIC-TOMISIC ET AL: "Hydrogen bonds in the crystal packings of mesalazine and mesalazine hydrochloride", JOURNAL OF MOLECULAR STRUCTURE, vol. 416, no. 1-3, 1 October 1997 (1997-10-01), pages 209-220, XP055113182, ISSN: 0022-2860, DOI: 10.1016/S0022-2860(97)00043-4

## Description

### FIELD OF THE INVENTION

The present invention relates to a process for the production of high-density mesalamine.

The present invention originates in the field of industrial processes for the production of active ingredients.

Specifically, the present invention relates to a process for the production of a crystalline form of mesalamine with a high bulk density, pharmaceutical compositions containing same and uses in the therapeutic field of the of form of high-density mesalamine obtained.

### BACKGROUND ART

5-Aminosalicylic acid or mesalamine (5-amino-2-hydroxybenzoic acid) is a molecule belonging to the class of salicylates, drugs provided with antiinflammatory activity (NSAIDs).

Mesalamine may mainly be applied in the treatment of inflammatory bowel diseases (IBD), such as Crohn's Disease and ulcerative colitis.

This latter is an inflammatory disease of the mucous membrane of the rectum and/or colon, localised in particular in the descending part. Ulcerative colitis is a disease whose etiology still remains unknown and which shows with ulcerations of the intestinal mucous membrane and hyperaemia. Crohn's disease, also known as regional enteritis, is also an inflammatory disease, but unlike ulcerative colitis, it is more widespread, being capable of affecting any part of the gastrointestinal tract, although it is mainly localised at the level of the ileum, the end part of the small intestine and the large intestine.

Crohn's disease is believed to be a condition with an autoimmune origin or with a main component of the autoimmune type, wherein the immune system attaches the mucous membrane of the gastrointestinal tract causing a chronic state of inflammation.

Currently there is no definitive pharmacological therapy yet and the treatment options are limited to controlling the symptoms, maintaining remission and preventing relapses.

The acute treatment of the disease is based on the use of antibiotic and antiinflammatory drugs, also corticosteroids. The prolonged use of corticosteroids, however, implies significant side effects, therefore, the administration of aminosalicylates, typically mesalamine, is preferred.

Mesalamine is administered in several pharmaceutical forms, mainly tablets coated with gastro-resistant films, delayed-delivery capsules, suppositories, microenemas, foams and rectal suspensions.

Generally, the mesalamine dosage required for treating IBD is high and may range from 400 to 1200 mg of active ingredient/day.

Consequently, currently there is a need for providing high-density 5-aminosalicylic acid which allows formulating high-dosage pharmaceutical preparations with formulation volumes lower than those currently commercially available.

The Patent Application US 2013/0225539A1 describes a process for the production of mesalamine having a bulk density from 300 g/l to 700 g/l, grain size distribution of X(10)= 1-30 µm; X(50)= 15-60 µm; X(90)= 35-220 µm and a tapped density from 510 to 900 g/l. This process comprises pouring a strongly basic alkaline solution in a 5-ASA solution having acidic pH with the formation of a bulk with large 5-ASA crystals in suspension which is wet ground in a homomixer, until reaching the desired particle size.

This process, however, has the drawback of providing a wet grinding step with homomixers of the Supraton or Ytron type, whose use takes place with high consumption of electric energy and management costs which make this technology not economically convenient.

Furthermore, this process requires a thorough monitoring of the crystallisation conditions both because heat develops in the area in which the strongly basic solution contacts the strongly acid solution of mesalamine and because the crystals that form in this exothermic reaction have large sizes.

These drawbacks make this process little attractive and competitive, therefore currently there is a need to provide a more economical and feasible technology to produce mesalamine with a high bulk density.

One of the objects of the present invention therefore consists in providing a process for the production of high-density 5-aminosalicylic acid to prepare pharmaceutical formulations with a high dosage of active ingredient and improved patient compliance.

Another object of the present invention consists in providing a process for the production of high-density mesalamine in a highly pure form, substantially solvent-free.

### SUMMARY OF THE INVENTION

The Applicant has found that by adopting specific reaction conditions it is possible to obtain mesalamine with very high apparent density, greater than that of mesalamine obtained with conventional precipitation techniques, in particular with values lower than 0.25 g/ml. Disclosed is a process for the production of mesalamine with a high bulk density in a highly pure form, achieving a progressive crystallisation at controlled pH. Further, there is provided a process for the production of mesalamine with a bulk density greater than or equal to 0.30 g/ml, substantially solvent-free, wherein the crystallisation is obtained by slowly pouring optionally in two or more portions, an acid solution of mesalamine hydrochloride in an aqueous solution buffered with a buffer system based on acetic acid or sodium acetate, so as to gradually increase the strongly acidic pH of the solution of mesalamine hydrochloride and promote the crystallisation of mesalamine with a high bulk density.

In particular, the process of the invention comprises the steps of
a) Dissolving mesalamine in an aqueous solution of HCI obtaining an acidic solution of mesalamine hydrochloride having a pH ≤ 2,
b) Adding the acid solution containing mesalamine HCl to an aqueous solution buffered with a buffer system based on acetic acid and sodium acetate having a pH from 3.5 to 4.5 to increase the pH value of the solution and promote the crystallisation of mesalamine with a bulk density greater than or equal to 0.30 g/ml,
c) Heating the melamine-based mass to make it reflux and maintaining it in these conditions until complete crystallisation.
d) Cooling and separating the high-density mesalamine obtained.

Some embodiments of the process of the invention are provided in the appended dependent claims. Typically, the mesalamine with a high bulk density obtained with the process of the invention is in a very pure crystalline form.

The mesalamine obtained in accordance with the process of the invention is in a highly pure form, substantially free of organic solvents, having a bulk density greater than or equal to 0.30 g/ml and suitable for being conveniently granulated. In accordance with certain embodiments the mesalamine of the disclosure has a bulk density of from 0.30 g/ml to 0.80 g/ml.

In accordance with some embodiments, the process for the production of mesalamine with a high bulk density of the invention may provide also the use of an organic solvent miscible with water.

The Applicant has in fact observed that by using an aprotic organic solvent mesalamine with a bulk density equal to or greater than 0.40 g/ml, conveniently between 0.40 and 0.80 g/ml, is obtained.

Mesalamine with a high bulk density, obtained in accordance with these embodiments, contains amounts in traces of organic solvent. Disclosed is also mesalamine substantially totally free of organic solvents having a bulk density greater than or equal to 0.30 g/m and pharmaceutical compositions containing the same. Also disclosed is mesalamine containing trace amounts of organic solvent having a bulk density greater than or equal to 0.40 g/ml and pharmaceutical compositions containing the same. Further disclosed is mesalamine with a bulk density greater than or equal to 0.30 g/ml obtained with the process of the invention for use in medicine.

### DETAILED DESCRIPTION OF THE INVENTION

The Applicant has found a process for the production of mesalamine (5-aminosalicylic acid) having a high density (bulk density) wherein the crystallisation is obtained by slowly pouring, optionally in two or more portions, an acid solution of mesalamine hydrochloride in an aqueous solution buffered with a buffer system based on acetic acid or sodium acetate. The invention is defined by the appended claims. In accordance with a first aspect, provided is a process for the production of mesalamine with a bulk density greater than or equal to 0.30 g/ml, substantially solvent-free, comprising slowly adding an acid solution of mesalamine hydrochloride to an aqueous solution buffered with a buffer system based on acetic acid and sodium acetate so as to gradually increase the pH value of the acid solution and promote crystallisation of mesalamine with a bulk density greater than or equal to 0.30 g/ml.

Therefore, the object of the invention is a process comprising the steps of
a) Dissolving mesalamine in an aqueous solution of HCl obtaining an acidic solution of mesalamine hydrochloride having a pH ≤ 2,
b) Adding the acid solution containing mesalamine HCl in an aqueous solution buffered with a buffer system based on acetic acid and sodium acetate having a pH from 3.5 to 4.5 to increase the pH value of the solution and promote the crystallisation of mesalamine with a bulk density greater than or equal to 0.30 g/ml,
c) Heating the melamine-based bulk to make it reflux, maintaining it in these conditions until crystallisation is complete,
d) Cooling and separating the high-density mesalamine obtained.

In accordance with certain embodiments the process for the production of mesalamine having a bulk density greater than or equal to 0.30 g/ml comprises the following steps:
i) adding mesalamine, hydrochloric acid and water to a reactor and heating to a temperature conveniently in the range between 30 and 90 °C in order to obtain a solution of mesalamine in the form of hydrochloride, conveniently with pH below 2.0, optionally adding bleaching coal to the solution and filtering to separate the solution,
ii) adding water, a weak acid, conveniently acetic acid, and sodium acetate to a second reactor, heating to complete solution and then bringing the temperature of the solution typically to 85-100 °C,
iii) pouring the acid solution of mesalamine in the solution of the second reactor keeping the temperature at 85-100 °C,
iv) cooling the resulting bulk and optionally spinning, rinsing with water and drying the resulting mesalamine.

Typically, the mesalamine obtained in accordance with certain embodiments of the invention is totally free of organic solvents and has a bulk density equal to or greater than 0.3 g/ml.

In some embodiments the mesalamine obtained has a bulk density of from 0.3 g/ml to 0.4 g/ml.

The process according to the invention has high production yields, typically greater than 90% and in some embodiments varying from 93 to 94%.

In step i) of the process of the invention mesalamine is dissolved in an aqueous solution of hydrochloric acid to provide a solution with a pH below 2.0. Typically distilled water, free of impurities which may interact with mesalamine and cause a staining of the solution, is used as a solvent.

In accordance with some embodiments in step i) an anti-oxidant substance is added, such as for example sodium metabisulfite to prevent mesalamine from oxidising.

In some embodiments the acid solution of mesalamine of step i) is supplemented with bleaching coal to adsorb any impurities still present and prevent the solution from staining.

The bleaching coal added to the acid solution of mesalamine is left in suspension and, after reacting, is separated, for example by filtration.

In some embodiments the hydrochloric acid added in step i) inside the first reactor is an aqueous solution with a concentration that may range from 30 to 40%. Conveniently, the components present in the first reactor are subjected to mixing by heating at a temperature comprised between 40 and 60°C until solution. According to some embodiments, the acetic acid used in step ii) of the process is provided as an aqueous solution with a concentration comprised between 70 and 90% by weight, typically equal to about 80% by weight. According to some embodiments, the solution of step ii) has an acidic pH, typically comprised between 4 and 5.5.

In accordance with some embodiments step ii) of the process comprises heating the mixture present in the second reactor at a temperature comprised between 40 and 60°C until complete solution. Later the temperature of the solution ii) is further heated, conveniently at a temperature of 85-100°C.

In accordance with certain embodiments in the second reactor there is also added an anti-oxidant agent, typically sodium metabisulfite.

In accordance with some embodiments in step ii) of the process in the second reactor there is added a chelating agent, EDTA in particular in the form of sodium salt, in order to prevent the mesalamine from oxidising or the formation of complexes with any ions present in the solution.

In step iii) of the process of the invention the acid solution of mesalamine prepared in step i) is poured in the solution of step ii), heated at temperatures close to reflux, typically in the range of 85-100°C.

Typically the addition of the acid solution containing mesalamine in the solution obtained in step ii) is carried out slowly; in certain embodiments the acid solution of mesalamine in poured in no less than one hour.

In some embodiments at the end of the pouring operations of the acid solution of mesalamine the temperature is kept at 85-100°C for a period of time of at least 10 minutes.

In the subsequent step iv) of the process the bulk obtained is conveniently cooled at a temperature of from 25 to 40°C and optionally centrifuged performing a rinse with water.

The bulk obtained in the previously described process is based on mesalamine with a high density, typically equal to or greater than 0.30 g/ml, conveniently totally free of organic solvents.

In certain embodiments the mesalamine bulk obtained is dried out for example by processing at a temperature comprised between 60 and 70°C.

In a second aspect, the invention relates to a variation of the process previously described, wherein a water-soluble aprotic organic solvent is added which is suitable for further increasing the bulk density of the mesalamine obtained.

In accordance with some embodiments therefore there is provided the addition, inside the second reactor, of a water-soluble aprotic organic solvent in step ii), in particular in a step following the formation of the solution.

A suitable aprotic organic solvent is a water-soluble ketone wherein the alkyl radicals comprise 1 to 6 carbons. Particularly suitable water-soluble ketones comprise acetone, 2-butanone, 2-pentanone, 3-pentanone, with acetone being the preferred ketone.

The mesalamine obtained using the aprotic organic solvent in the process of the invention has a purity of the pharmaceutical grade and contains only trace amounts of the organic solvent.

In particular, the mesalamine obtained using a water-soluble aprotic organic solvent has a bulk density greater than or equal to 0.4 g/ml and in some embodiments has an amount of aprotic organic solvent lower than 1000 ppm.

In some embodiments the mesalamine obtained using the water-soluble aprotic organic solvent, in particular acetone is comprised from 0.4 to 0.6 g/ml and preferably from 0.45 to 0.55 g/ml. The bulk density is measured using methods known to those skilled in the art for example such as those described in the United States Pharmacopeia (USP) for example USP 27, Vol.1 pages 226-227 1 May 2009-30 April 2010, Bulk density 616, Method 1.

One of the advantages of the process of the invention consists in obtaining mesalamine with a bulk density greater than or equal to 0.30 g/ml with high production yields, typically greater than 92%.

Another advantage consists in the possibility of obtaining 5-aminosalicylic acid with a bulk density greater than or equal to 0.30 g/ml in a crystalline form essentially free of impurities and solvents.

Another advantage consists in the possibility of modulating and increasing the degree of density of active ingredient by adding in the production step a water-soluble aprotic organic solvent, typically acetone, and obtaining a highly pure product wherein the solvent is present in trace amounts, for example in an amount of less than 1000 ppm.

Within the scope of the present invention, the term reactor is to be intended in a broad sense as a suitable environment wherein the two solutions to be mixed are prepared to obtain mesalamine in the pure form.

Within the scope of the present invention, the terms mesalamine, mesalazine and 5-ASA indicate the same substance and are to be considered as equivalent to each other.

Typically the mesalamine obtained by means of the process of the invention has a purity of the pharmaceutical grade and finds application in the production of medicaments.

In accordance with a further aspect, disclosed is mesalamine having a bulk density greater than or equal to 0.30 g/ml, conveniently comprised between 0.30 and 0.40 g/ml obtained with the process of the invention.

In accordance with another aspect, disclosed is mesalamine with a content of an aprotic organic solvent, in particular acetone lower than 1000 ppm and having a bulk density greater than or equal to 0.40 g/ml, conveniently comprised between 0.40 and 0.60 g/ml obtained with the process of the invention.

In accordance with another aspect also disclosed is a pharmaceutical composition comprising a pharmaceutically active amount of highly pure mesalamine obtained with a process according to any of the embodiments previously described, and a pharmaceutically acceptable carrier and/or excipient.

Pharmaceutically acceptable carriers and excipients comprise the substances generally used in the production techniques of the pharmaceutical and medical device industry.

The present invention claims priority over the Italian patent application MI2013A001497 of 10 September 2013. The present invention shall now be described with reference to the following examples which are provided for illustration purposes and shall not be intended as limiting of the scope of the present invention.

### EXAMPLES

### EXAMPLE 1

In a first reactor, there are loaded:
300 kg mesalamine
or alternatively
300 kg crude mesalamine (use the amount equivalent of wet product)
2000 kg deionised water
4,5 kg sodium metabisulfite
254 kg 37% hydrochloric acid
Heat the bulk at 40-60°C until complete solution
Add
5.5 kg bleaching coal
Filter the suspension and put aside

In another reactor, there are loaded:
1650 kg deionised water
2.25 kg disodium EDTA
225 kg sodium acetate
9,0 kg sodium metabisulfite
45 kg 80% acetic acid
Heat at 40-60°C until complete solution
Take the temperature of the solution to 85-100°C then slowly pour (pour the first quarter of the solution in about 30', wait for precipitation, then pour the remaining solution in further about 30') all the acid solution containing the product Then keep at 85-100°C for at least 10'
Cool the bulk at 25-40°C then centrifuge washing with
3000 kg deionised water
Dry at 60-70°C
About 280 kg solvent-free heavy mesalamine is obtained (bulk density greater than or equal to 0.30 g/ml - free of organic solvents)
Yield: 93.3%

### EXAMPLE 2

In a first reactor, there are loaded:
15.0 kg mesalamine
or alternatively
300 kg crude mesalamine (use the amount equivalent of wet product)
100 kg deionised water
12.7 kg 37% hydrochloric acid
Heat the bulk at 40-60°C until complete solution
Add
0.275 kg bleaching coal
Filter the suspension and put aside

In a second reactor, there are loaded:
82.5 kg deionised water
0,11 kg disodium EDTA
11,25 kg sodium acetate
0.45 kg sodium metabisulfite
2.25 kg 80% acetic acid
Heat at 40-60°C until complete solution, then add
8.3 kg acetone
Take the temperature of the solution to 75-85°C then slowly pour (pour the first quarter of the solution in about 30', wait for precipitation, then pour the remaining solution in further about 30') all the acid solution containing the product Then keep at 75-85°C for at least 10'
Cool the bulk at 25-40°C then centrifuge washing with
150 kg deionised water
Dry at 60-70°C
About 14.0 kg heavy mesalamine is obtained (bulk density greater than or equal to 0.40 g/ml - residual acetone lower than 1000 ppm)
Yield: 93.3%

## Claims

1. A process for the production of mesalamine with a bulk density greater than or equal to 0.30 g/ml, substantially solvent-free comprising the steps of
a) Dissolving mesalamine in an aqueous solution of HCI obtaining an acidic solution of mesalamine hydrochloride having a pH ≤ 2,
b) Adding the acid solution of mesalamine HCI to an aqueous solution buffered with a buffer system based on acetic acid and sodium acetate having a pH from 3.5 to 4.5 to increase the pH value of the solution and promote the crystallisation of mesalamine with a bulk density greater than or equal to 0.30 g/ml,
c) Heating the melamine-based mass to make it reflux and maintaining it in these conditions until complete crystallisation.
d) Cooling and separating the high-density mesalamine obtained.

2. A process according to claim 1, wherein step b) comprises increasing the temperature of the buffered aqueous solution to reflux.

3. A process according to claim 1 or 2, wherein step b) comprises slowly adding the acid solution containing mesalamine hydrochloride divided into two or more portions to the buffered aqueous solution.

4. A process according to any one of claims 1-3, wherein the separation of mesalamine in step d) is carried out by centrifugation.

5. A process according to any one of claims 1-4, wherein after separation, the melamine-based bulk is washed with water, dried and optionally dried at 60-70°C.

6. A process according to any of claims 1-5, wherein step a) of mesalamine dissolution is carried out under stirring of the HCI solution at a temperature comprised between 40 and 60°C.

7. A process according to any one of the preceding claims 1-6, comprising adding sodium metabisulfite and/or bleaching coal to the HCI aqueous solution of step a) and optionally adding EDTA or a sodium salt thereof to the buffer aqueous solution of step b).

8. A process according to any one of claims 1-7, wherein in step b) the buffered aqueous solution has a temperature comprised between 40 and 60° and the temperature of said solution at 85-100 °C.

9. A process according to any one of the preceding claims 1-8, wherein after completely pouring the acid solution of mesalamine hydrochloride in the buffer aqueous solution, it is kept at a temperature of 85-100 °C for at least 10 minutes.

10. A process according to any one of the preceding claims 1-9, wherein step b) comprises adding an aprotic organic solvent to obtain heavy mesalamine having a bulk density greater than or equal to 0.40 g/ml and residual organic solvent content of less than 1000 ppm.

## Patentansprüche

1. Verfahren zur Herstellung von Mesalazin mit einer Volumendichte von größer als oder gleich 0,30 g/ml, im Wesentlichen lösungsmittelfrei, umfassend die Schritte
a) Auflösen von Mesalazin in einer wässrigen Lösung von HCl unter Erhalt einer sauren Lösung von Mesalazinhydrochlorid mit einem pH ≤ 2,
b) Zugeben der Säurelösung von Mesalazin-HCI zu einer wässrigen Lösung, die mit einem Puffersystem auf der Basis von Essigsäure und Natriumacetat mit einem pH von 3,5 bis 4,5 gepuffert ist, um den pH-Wert der Lösung zu erhöhen und die Kristallisation vo Mesalazin mit einer Volumendichte von größer als oder gleich 0,3 g/ml zu fördern,
c) Erwärmen der melaminbasierten Masse, um sie refluxieren zu lassen und Halten derselben unter diesen Bedingungen bis zur vollständigen Kristallisation,
d) Kühlen und Abscheiden des erhaltenen hochdichten Mesalazins.

2. Verfahren nach Anspruch 1, wobei Schritt b) ein Erhöhen der Temperatur der gepufferten wässrigen Lösung bis zum Rückfluss umfasst.

3. Verfahren nach Anspruch 1 oder 2, wobei Schritt b) ein langsames Zugeben der Säurelösung, die Mesalazinhydrochlorid enthält, das in zwei oder mehr Anteile unterteilt ist, zu der gepufferten wässrigen Lösung umfasst.

4. Verfahren nach einem der Ansprüche 1 bis 3, wobei die Abscheidung des Mesalazins in Schritt d) durch Zentrifugieren ausgeführt wird.

5. Verfahren nach einem der Ansprüche 1 bis 4, wobei nach der Abscheidung die melaminbasierte Masse mit Wasser gewaschen, getrocknet und optional bei 60 - 70°C getrocknet wird.

6. Verfahren nach einem der Ansprüche 1 bis 5, wobei Schritt a) der Mesalazinauflösung unter Rühren der HCl-Lösung bei einer Temperatur zwischen 40 und 60°C ausgeführt wird.

7. Verfahren nach einem der vorhergehenden Ansprüche 1 - 6, das das Zugeben von Natriummetabisulfit und/oder Bleichkohle zu der wässrigen HCl-Lösung von Schritt a) und optional das Zugeben von EDTA oder eines Natriumsalzes davon zu der wässrigen Pufferlösung von Schritt b) umfasst.

8. Verfahren nach einem der Ansprüche 1 - 7, wobei in Schritt b) die gepufferte wässrige Lösung eine Temperatur zwischen 40 und 60°C aufweist und die Temperatur der Lösung bei 85 - 100°C liegt.

9. Verfahren nach einem der vorhergehenden Ansprüche 1 - 8, wobei nach dem vollständigen Eingießen der wässrigen Lösung von Mesalazinhydrochlorid in die wässrige Pufferlösung diese für mindestens 10 Minuten bei einer Temperatur von 85 - 100°C gehalten wird.

10. Verfahren nach einem der vorhergehenden Ansprüche 1 bis 9, wobei Schritt b) das Zugeben eines aprotischen organischen Lösungsmittels umfasst, um schweres Mesalazin mit einer Volumendichte von größer als oder gleich 0,40 g/ml und einen organischen Restlösemittelgehalt von weniger als 1000 ppm zu erhalten.

## Revendications

1. Procédé de production de mésalamine avec une masse volumique apparente supérieure ou égale à 0,30 g/ml, sensiblement sans solvant, comprenant les étapes consistant à :
a) dissoudre la mésalamine dans une solution aqueuse de HCl en obtenant une solution acide de chlorhydrate de mésalamine de pH ≤ 2,
b) ajouter la solution acide de mésalamine-HCl à une solution aqueuse tamponnée avec un système de tampon à base d'acide acétique et d'acétate de sodium ayant un pH de 3,5 à 4,5 pour augmenter la valeur de pH de la solution et favoriser la cristallisation de la mésalamine avec une masse volumique apparente supérieure ou égale à 0,30 g/ml,
c) chauffer la masse à base de mélamine pour la faire refluer et la maintenir dans ces conditions jusqu'à une cristallisation complète.
d) refroidir et séparer la mésalamine haute densité obtenue.

2. Procédé selon la revendication 1, dans lequel l'étape b) comprend l'augmentation de la température de la solution aqueuse tamponnée jusqu'au reflux.

3. Procédé selon la revendication 1 ou 2, dans lequel l'étape b) comprend l'addition lente de la solution acide contenant du chlorhydrate de mésalamine divisée en deux portions ou plus à la solution aqueuse tamponnée.

4. Procédé selon l'une quelconque des revendications 1 à 3, dans lequel la séparation de la mésalamine dans l'étape d) est réalisée par centrifugation.

5. Procédé selon l'une quelconque des revendications 1 à 4, dans lequel après séparation, la masse à base de mélamine est lavée avec de l'eau, séchée et éventuellement séchée entre 60 et 70°C.

6. Procédé selon l'une quelconque des revendications 1 à 5, dans lequel l'étape a) de dissolution de mésalamine est effectuée sous agitation de la solution de HCl à une température comprise entre 40 et 60°C.

7. Procédé selon l'une quelconque des revendications 1 à 6, comprenant l'addition de métabisulfite de sodium et/ou de charbon blanchissant à la solution aqueuse de HCl de l'étape a) et éventuellement l'addition d'EDTA ou d'un sel de sodium de celui-ci à la solution aqueuse tampon de l'étape b).

8. Procédé selon l'une quelconque des revendications 1 à 7, dans lequel dans l'étape b), la solution aqueuse tamponnée a une température comprise entre 40 et 60°, et la température de ladite solution entre 85 et 100°C.

9. Procédé selon l'une quelconque des revendications 1 à 8, dans lequel, après avoir versé complètement la solution acide de chlorhydrate de mésalamine dans la solution aqueuse tampon, elle est maintenue à une température entre 85 et 100°C pendant au moins 10 minutes.

10. Procédé selon l'une quelconque des revendications 1 à 9, dans lequel l'étape b) comprend l'addition d'un solvant organique aprotique pour obtenir une mésalamine lourde ayant une masse volumique apparente supérieure ou égale à 0,40 g/ml et une teneur en solvant organique résiduel de moins que 1 000 ppm.
